# EUROPEAN PATENT APPLICATION

(11) **EP 4 635 423 A1**
(43) Date of publication of application: **22.10.2025**
(21) Application number: 24738553.7
(22) Date of filing: 05.01.2024
(51) Int. Cl.: A61B 8/12, A61B 8/00

(54) **INTRAVASCULAR ULTRASOUND IMAGING METHOD AND APPARATUS, COMPUTER DEVICE AND STORAGE MEDIUM**

(30) Priority: 06.01.2023 CN 202310015257
(71) Applicant: Microimaging (Shenzhen) Medical Equipment Co., Ltd, Shenzhen, Guangdong 518110 (CN)
(72) Inventor: HE, Qing, Shenzhen, Guangdong 518110 (CN); HE, Zhihua, Shenzhen, Guangdong 518110 (CN); CHEN, Haiping, Shenzhen, Guangdong 518110 (CN); YANG, Liuen, Shenzhen, Guangdong 518110 (CN); LIU, Xia, Shenzhen, Guangdong 518110 (CN)
(74) Representative: Murgitroyd & Company
(86) International application number: PCT/CN2024/070750
(87) International publication number: WO 2024/146622

(57) **Abstract**

Disclosed are an intravascular ultrasound imaging method and apparatus, a computer device, and a storage medium. The method includes: (S202) acquiring a main scan pulse echo corresponding to a main scan pulse; (S204) extracting a waveform feature of the main scan pulse echo, obtaining waveform feature-supplementary pulse type association information, and according to the association information, determining a target supplementary pulse type corresponding to the waveform feature of the main scan pulse echo; (S206) according to the target supplementary pulse type, determining at least one supplementary scan pulse, and acquiring supplementary scan pulse echoes respectively corresponding to the supplementary scan pulses; and (S208) according to the main scan pulse echo and the at least one supplementary scan pulse echo, obtaining an intravascular ultrasound imaging result.

## Description

### RELATED APPLICATIONS

This application claims priority to Chinese Patent Application No. 202310015257.3, entitled "Intravascular Ultrasound Imaging Method and Apparatus, Computer Device and Storage Medium", filed on January 6, 2023, which is incorporated herein by reference in its entirety.

### TECHNICAL FIELD

The present application relates to the field of intravascular ultrasound technologies, and in particular, to an intravascular ultrasound imaging method and apparatus, a computer device, a storage medium and a computer program product.

### BACKGROUND

Intravascular ultrasound imaging, which is also called an intravascular ultrasound (i.e., IVUS) technology, is a technology in which a miniature ultrasound probe is mounted at a front end of a catheter, a tissue structure of a blood vessel is probed by inserting the catheter into the blood vessel using a professional technology, and the intravascular ultrasound imaging is a relatively effective, direct and high-quality ultrasound diagnostic technology at the present stage. Since the IVUS technology has a higher identification rate for components in the blood vessel and can clearly display tissue structure information of a blood vessel wall, the IVUS technology is gradually recommended by various medical guidelines at present and is more and more widely applied in interventional catheter laboratories. In the aspect of cardiovascular disease diagnosis, with the IVUS technology, not only a size, shape and vessel wall structure of a lumen can be learnt, but also a sectional area of the vessel lumen can be precisely measured, and lesions such as vessel calcification, fibrosis and lipid nuclei can be identified.

Currently common commercial IVUS systems have scan frequencies generally concentrated at 20MHz to 60MHz and axial resolutions concentrated at 22um to 60um. Generally speaking, the higher the scan frequency of the IVUS system, the better the axial resolution, which helps identify some small volume lesion features such as thin-cap fibroatheroma in an IVUS image. However, increasing the scan frequency also reduces penetrability of an ultrasound excitation signal to reduce a scan depth, and meanwhile reduces a resolution of blood and non-blood tissue, so that it is difficult to distinguish and identify the blood lumen wall in the blood-filled lumen.

The current IVUS system has a single scan frequency, cannot realize precise imaging in a special scenario and has the problem of low applicability.

### SUMMARY

Based on this, in order to solve the above technical problem, there is provided an intravascular ultrasound imaging method and apparatus, a computer device, a computer-readable storage medium, and a computer program product capable of improving imaging applicability.

In a first aspect, the present application provides an intravascular ultrasound imaging method. The method includes:
acquiring a main scan pulse echo corresponding to a main scan pulse;
extracting a waveform feature of the main scan pulse echo, obtaining waveform feature-supplementary pulse type association information, and according to the association information, determining a target supplementary pulse type corresponding to the waveform feature of the main scan pulse echo;
according to the target supplementary pulse type, determining at least one supplementary scan pulse, and acquiring supplementary scan pulse echoes respectively corresponding to the supplementary scan pulses; and
according to the main scan pulse echo and the at least one supplementary scan pulse echo, obtaining an intravascular ultrasound imaging result.

In an embodiment, acquiring the main scan pulse echo corresponding to the main scan pulse includes:
emitting the main scan pulse to a target position, and receiving the returned main scan pulse echo.

In an embodiment, acquiring the supplementary scan pulse echoes respectively corresponding to the supplementary scan pulses includes:
emitting the at least one supplementary scan pulse to the target position at one time, and receiving the returned supplementary scan pulse echoes in sequence to obtain the at least one supplementary scan pulse echo.

In an embodiment, according to the main scan pulse echo and the at least one supplementary scan pulse echo, obtaining the intravascular ultrasound imaging result includes:
taking the main scan pulse echo and the at least one supplementary scan pulse echo as a group of single-scan data corresponding to the target position;
in a scan period, determining a plurality of different target positions and acquiring the group of single-scan data corresponding to each target position; and
obtaining the intravascular ultrasound imaging result according to the multiple groups of single-scan data.

In an embodiment, obtaining the intravascular ultrasound imaging result according to the multiple groups of single-scan data includes:
respectively performing information fusion on the main scan pulse echo and the at least one supplementary scan pulse echo in the same group of single-scan data to obtain a plurality of fusion echo signals; and
performing image reconstruction according to the plurality of fusion echo signals to obtain the intravascular ultrasound imaging result.

In an embodiment, obtaining the intravascular ultrasound imaging result according to the multiple groups of single-scan data further includes:
performing image reconstruction according to the main scan pulse echo in each group of single-scan data to obtain a first imaging result;
performing image reconstruction according to the at least one supplementary scan pulse echo in each group of single-scan data to obtain at least one second imaging result; and
performing image fusion according to the first imaging result and the at least one second imaging result to obtain the intravascular ultrasound imaging result.

In an embodiment, according to the main scan pulse echo and the at least one supplementary scan pulse echo, obtaining the intravascular ultrasound imaging result further includes:
acquiring a signal frequency of the main scan pulse echo;
determining preprocessing parameters according to the signal frequency, and performing ultrasonic signal preprocessing on the main scan pulse echo and the at least one supplementary scan pulse echo by adopting the preprocessing parameters; and
obtaining the intravascular ultrasound imaging result according to the main scan pulse echo and the at least one supplementary scan pulse echo after the ultrasonic signal preprocessing.

In an embodiment, obtaining the waveform feature-supplementary pulse type association information includes:
acquiring a plurality of vascular tissue features related to intravascular ultrasound, and determining a supplementary pulse type corresponding to each vascular tissue feature;
acquiring a sample scan pulse echo corresponding to each vascular tissue feature, and extracting a sample waveform feature corresponding to each vascular tissue feature from each sample scan pulse echo; and
acquiring the waveform feature-supplementary pulse type association information according to the sample waveform feature and the supplementary pulse type corresponding to the same vascular tissue feature.

In an embodiment, extracting the waveform feature of the main scan pulse echo, obtaining the waveform feature-supplementary pulse type association information, and according to the association information, determining the target supplementary pulse type corresponding to the waveform feature of the main scan pulse echo includes:
identifying the waveform feature to obtain an identification result; and
if the identification result meets a supplementary pulse condition, determining the target supplementary pulse type corresponding to the waveform feature according to the waveform feature-supplementary pulse type association information.

In an embodiment, the method further includes:
if the identification result does not meet the supplementary pulse condition, obtaining the intravascular ultrasound imaging result according to the main scan pulse echo.

In an embodiment, a scan frequency of the supplementary scan pulse is different from a scan frequency of the main scan pulse.

In an embodiment, the preprocessing parameters include a tap coefficient of an FIR band-pass filter and a demodulation coefficient in quadrature demodulation.

In a second aspect, the present application further provides an intravascular ultrasound imaging apparatus. The apparatus includes:
a first scan module configured to acquire a main scan pulse echo corresponding to a main scan pulse;
an echo processing module configured to extract a waveform feature of the main scan pulse echo, obtain waveform feature-supplementary pulse type association information, and according to the association information, determine a target supplementary pulse type corresponding to the waveform feature of the main scan pulse echo;
a second scan module further configured to, according to the target supplementary pulse type, determine at least one supplementary scan pulse, and acquire supplementary scan pulse echoes respectively corresponding to the supplementary scan pulses; and
an ultrasound imaging module configured to, according to the main scan pulse echo and the at least one supplementary scan pulse echo, obtain an intravascular ultrasound imaging result.

In a third aspect, the present application further provides a computer device. The computer device includes a memory and a processor. The memory stores a computer program, and the processor, when executing the computer program, implements the following steps:
acquiring a main scan pulse echo corresponding to a main scan pulse;
extracting a waveform feature of the main scan pulse echo, obtaining waveform feature-supplementary pulse type association information, and according to the association information, determining a target supplementary pulse type corresponding to the waveform feature of the main scan pulse echo;
according to the target supplementary pulse type, determining at least one supplementary scan pulse, and acquiring supplementary scan pulse echoes respectively corresponding to the supplementary scan pulses; and
according to the main scan pulse echo and the at least one supplementary scan pulse echo, obtaining an intravascular ultrasound imaging result.

In a fourth aspect, the present application further provides a computer-readable storage medium. The computer-readable storage medium has a computer program stored thereon, and the computer program, when executed by a processor, implements the following steps:
acquiring a main scan pulse echo corresponding to a main scan pulse;
extracting a waveform feature of the main scan pulse echo, obtaining waveform feature-supplementary pulse type association information, and according to the association information, determining a target supplementary pulse type corresponding to the waveform feature of the main scan pulse echo;
according to the target supplementary pulse type, determining at least one supplementary scan pulse, and acquiring supplementary scan pulse echoes respectively corresponding to the supplementary scan pulses; and
according to the main scan pulse echo and the at least one supplementary scan pulse echo, obtaining an intravascular ultrasound imaging result.

In a fifth aspect, the present application further provides a computer program product. The computer program product includes a computer program which, when executed by a processor, implements the following steps:
acquiring a main scan pulse echo corresponding to a main scan pulse;
extracting a waveform feature of the main scan pulse echo, obtaining waveform feature-supplementary pulse type association information, and according to the association information, determining a target supplementary pulse type corresponding to the waveform feature of the main scan pulse echo;
according to the target supplementary pulse type, determining at least one supplementary scan pulse, and acquiring supplementary scan pulse echoes respectively corresponding to the supplementary scan pulses; and
according to the main scan pulse echo and the at least one supplementary scan pulse echo, obtaining an intravascular ultrasound imaging result.

In the intravascular ultrasound imaging method and apparatus, the computer device, the storage medium and the computer program product described above, the main scan pulse echo corresponding to the main scan pulse is acquired; the waveform feature of the main scan pulse echo is extracted, the waveform feature-supplementary pulse type association information is obtained, and according to the association information, the target supplementary pulse type corresponding to the waveform feature of the main scan pulse echo is determined; according to the target supplementary pulse type, the at least one supplementary scan pulse is determined, and the supplementary scan pulse echoes respectively corresponding to the supplementary scan pulses are acquired; and according to the main scan pulse echo and the at least one supplementary scan pulse echo, the intravascular ultrasound imaging result is obtained. The supplementary scan pulse can have the scan frequency different from that of the main scan pulse, and ultrasound imaging is performed in combination with multi-frequency scan pulses, so that clear and complete images can be ensured to be obtained in different intravascular scenarios, and applicability of intravascular ultrasound imaging is improved.

### BRIEF DESCRIPTION OF THE DRAWINGS

In order to illustrate the technical solutions in the embodiments of the present application or the related art more clearly, the drawings required for describing the embodiments or the related art will be described briefly. Apparently, the following described drawings are merely for some embodiments of the present application, and other drawings can be derived from these drawings by those of ordinary skill in the art without any creative effort.
FIG. 1 is a view of an application environment of an intravascular ultrasound imaging method according to an embodiment of the present application.
FIG. 2 is a schematic flow diagram of the intravascular ultrasound imaging method according to an embodiment of the present application.
FIG. 3 is a schematic diagram of a target position and a waveform feature in an embodiment of the present application.
FIG. 4 is a schematic diagram of pulse composition for each scan in an embodiment of the present application.
FIG. 5 is a schematic flow diagram of a first image reconstruction manner in an embodiment of the present application.
FIG. 6 is a schematic flow diagram of a second image reconstruction manner in an embodiment of the present application.
FIG. 7 is a schematic flow diagram of preprocessing in an embodiment of the present application.
FIG. 8 is a schematic flow diagram of an intravascular ultrasound imaging system in an embodiment of the present application.
FIG. 9 is a structural block diagram of an intravascular ultrasound imaging apparatus according to an embodiment of the present application.
FIG. 10 is an internal structure diagram of a computer device according to an embodiment of the present application.

### DETAILED DESCRIPTION

The present application will be described in further detail below with reference to the accompanying drawings and embodiments in order to make the objects, technical solutions, and advantages of the present application more clear. It should be understood that the specific embodiments described herein are only for explaining the present application, and not intended to limit the present application.

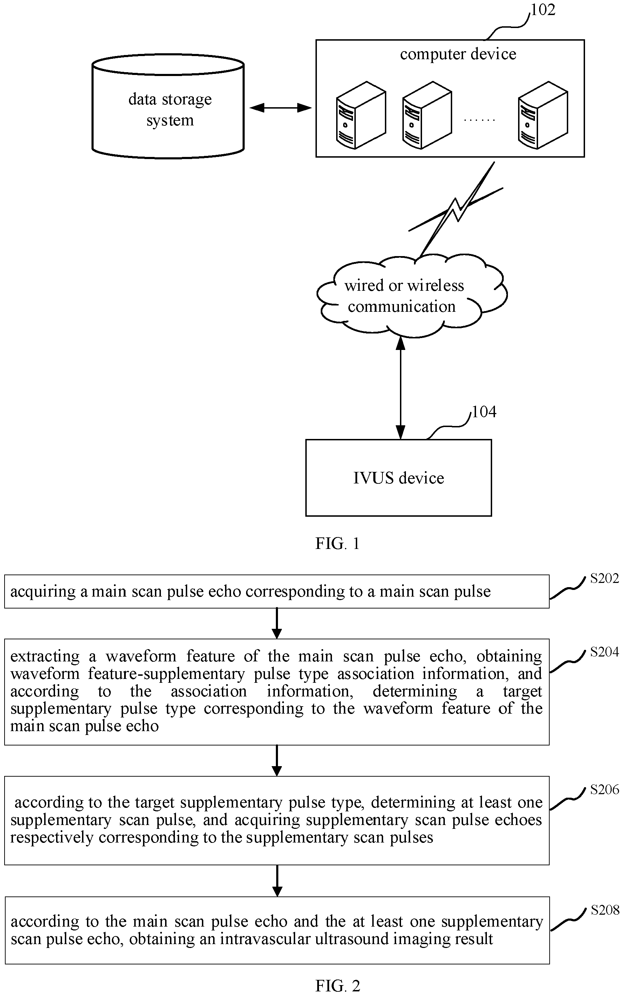

An intravascular ultrasound imaging method according to embodiments of the present application can be applied to an application environment shown in FIG. 1. A computer device 102 is communicated with an IVUS device 104 via a wired or wireless network. A data storage system may store data required to be processed by the computer device 102. The data storage system may be integrated on the computer device 102, or may be arranged on a cloud or another network server. The computer device 102 may be a terminal or a server. The terminal may be, but is not limited to, various personal computers, notebook computers, smart phones, tablet computers, Internet of Things devices and portable wearable devices. The portable wearable device can be a smart watch, a smart bracelet, a head-mounted device, or the like. The server may be a stand-alone server or may be implemented by a server cluster composed of a plurality of servers.

In an embodiment, as shown in FIG. 2, an intravascular ultrasound imaging method is provided, the description is given with the example where the method is applied to the computer device 102 in FIG. 1, and the method includes the following steps S202 to S208:

Step S202: acquiring a main scan pulse echo corresponding to a main scan pulse.

In a specific embodiment, the computer device controls the IVUS device to emit a main scan pulse to a current target position and receives a returned main scan pulse echo through the IVUS device as first partial data for scanning of the current target position.

The target position refers to a scanning position where the IVUS device performs one scan. Generally, the IVUS device generates one ultrasound image frame corresponding to one scan period, multiple scans are required in one scan period, and the scanning positions thereof are different. That is, one ultrasound image frame corresponds to multiple different target positions.

Step S204: extracting a waveform feature of the main scan pulse echo, obtaining waveform feature-supplementary pulse type association information, and according to the association information, determining a target supplementary pulse type corresponding to the waveform feature of the main scan pulse echo.

The waveform feature is used to characterize a vascular tissue feature at the target position. The vascular tissue features include, but are not limited to, a distance between vascular tissue and an IVUS probe, a thickness of the vascular tissue, or the like. Supplementary pulse types include, but are not limited to, a high frequency pulse and a low frequency pulse.

Optionally, the computer device identifies a critical wave band from the main scan pulse echo, identifies the waveform feature of the main scan pulse echo from the critical wave band, and determines the target supplementary pulse type corresponding to the main scan pulse echo received in this scan according to the preset waveform feature-supplementary pulse type association information.

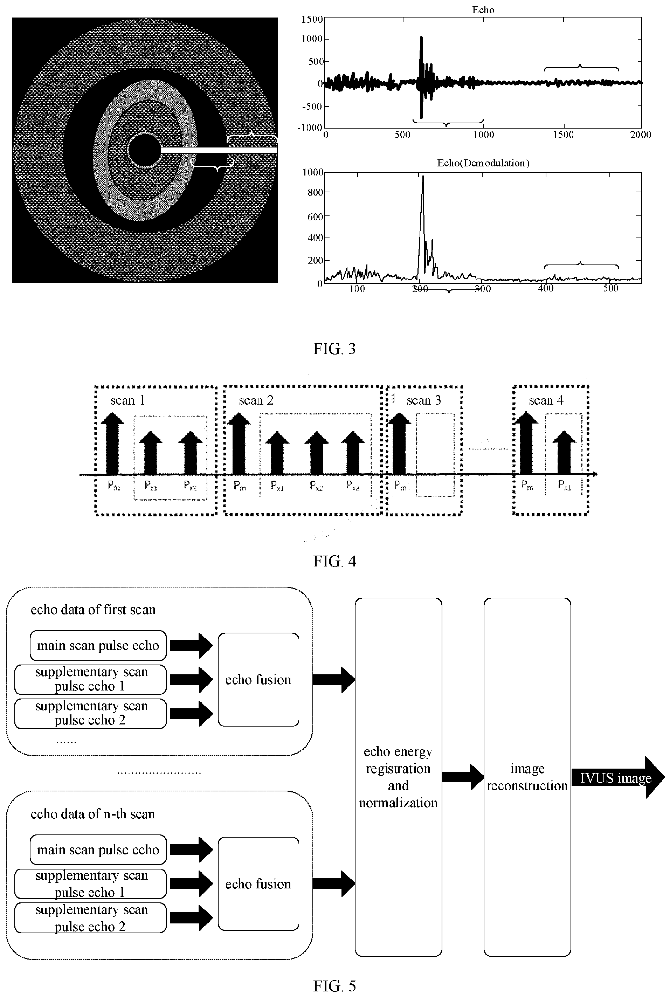

Specifically, after the main scan pulse is emitted, the IVUS device receives the main scan pulse echo and performs signal processing, and meanwhile transmits the main scan pulse echo to the computer device. The computer device identifies vascular tissue distribution information in real time through the main scan pulse echo. The vascular tissue distribution information is equivalent to the waveform feature. A decision as to whether a supplementary scan pulse needs to be emitted and what kind of supplementary pulse needs to be transmitted in the current scan period is then made. As shown in FIG. 3, the white bold line position in the left drawing is the target position of one scan, the waveforms in the right drawings correspond to echo signals at the white bold line position in the left drawing, the upper right part shows an original echo signal and the lower right part shows a demodulated echo signal. In the drawing, the three lower braces correspond to one critical wave band of the echo signal of vascular intima tissue at the same position, and the three upper braces correspond to another critical wave band of the echo signal of the vascular intima tissue at the same position. It can be seen that the echo waveform near the vascular intima tissue is significantly different from the echo waveforms at other positions. Therefore, a time position of a high-energy section in the echo can be determined using a time domain signal detection method, so as to obtain the distance between the vascular tissue and the probe and the thickness of the vascular tissue. After the identification is finished, the corresponding target supplementary pulse type is determined according to the waveform feature-supplementary pulse type association information. It should be noted that besides the above processing method based on the time domain waveform, the identification of the vascular tissue distribution information can be performed in other transform domains (such as a frequency domain, a time-frequency domain, and a wavelet domain). For the specific identification processing method, a pattern identification or deep learning method may be combined to perform waveform feature identification on the main scan pulse echo to correspond to different processing strategies, which is not limited in this embodiment.

Step S206: according to the target supplementary pulse type, determining at least one supplementary scan pulse, and acquiring supplementary scan pulse echoes respectively corresponding to the supplementary scan pulses.

In a specific embodiment, one or more supplementary scan pulses may be pre-stored in the computer device for each supplementary pulse type. The number of the supplementary scan pulses corresponding to each supplementary pulse type and the waveform of each supplementary scan pulse may be set in advance. After determining the target supplementary pulse type for one scan, the computer device controls the IVUS device to emit the at least one supplementary scan pulse to the target position at one time, and sequentially receive the returned supplementary scan pulse echoes, and the supplementary scan pulse echoes are in one-to-one correspondence to the supplementary scan pulses, so that the at least one supplementary scan pulse echo is obtained as second partial data for scanning the current target position.

In a feasible implementation, the IVUS device may use the same number of supplementary scan pulses per scan in one scan period.

Step S208: according to the main scan pulse echo and the at least one supplementary scan pulse echo, obtaining an intravascular ultrasound imaging result.

In a specific embodiment, the computer device stores the main scan pulse echo (as the first partial data) and the at least one supplementary scan pulse echo (as the second partial data) as a group of single-scan data obtained by scanning one target location. A next target position is then determined, and the second target position is scanned in the same manner to obtain a second group of single-scan data. The steps are repeated until all the target positions in one scan period are scanned, and multiple groups of single-scan data corresponding to the target positions respectively are obtained. Usually, the single-scan data is one-dimensional data, and the computer device finally performs image reconstruction according to the multiple groups of single-scan data to obtain a two-dimensional image, i.e., the intravascular ultrasound imaging result.

In the intravascular ultrasound imaging method described above, the main scan pulse echo corresponding to the main scan pulse is acquired; the waveform feature of the main scan pulse echo is extracted, the waveform feature-supplementary pulse type association information is obtained, and according to the association information, the target supplementary pulse type corresponding to the waveform feature of the main scan pulse echo is determined; according to the target supplementary pulse type, the at least one supplementary scan pulse is determined, and the supplementary scan pulse echoes respectively corresponding to the supplementary scan pulses are acquired; and according to the main scan pulse echo and the at least one supplementary scan pulse echo, the intravascular ultrasound imaging result is obtained. The supplementary scan pulse can have a scan frequency different from that of the main scan pulse, and ultrasound imaging is performed in combination with multi-frequency scan pulses, so that clear and complete images can be ensured to be obtained in different intravascular scenarios, and applicability of intravascular ultrasound imaging is improved.

In an embodiment, the step of according to the main scan pulse echo and the at least one supplementary scan pulse echo, obtaining an intravascular ultrasound imaging result may include: taking the main scan pulse echo and the at least one supplementary scan pulse echo as a group of single-scan data corresponding to the target position; in a scan period, determining a plurality of different target positions and acquiring the group of single-scan data corresponding to each target position; and obtaining the intravascular ultrasound imaging result according to the multiple groups of single-scan data.

In an embodiment, as shown in FIG. 4, intervals of the scans are the same, and at the beginning of one scan, one main scan pulse Pₘ is emitted first. After the main scan pulse is emitted out, the computer device may identify the echo generated by the pulse in real time, and according to the identification result, confirm whether a supplementary waveform needs to be emitted and what kind of supplementary scan pulse (for example, Pₓ₁, Pₓ₂, etc.) needs to be emitted in the present scan period. Since the scanning interval is fixed, transmission and corresponding echo reception of the supplementary scan pulse required to be emitted in this scan need to be completed within the scanning interval.

The main scan pulse Pₘ performs a preliminary scan on the target tissue. The echo generated by the main scan pulse is not only used for imaging, but also input into an echo discrimination module in real time. The echo discrimination module confirms a strategy for subsequent supplementary pulse emission according to the echo. A low frequency band of a scan frequency range supportable by a system can be selected as the main scan pulse Pₘ, so as to acquire more tissue profile and distribution information. In another embodiment, it can also be considered to use a middle frequency as Pₘ, so that the Pₘ pulse has higher universality for different vascular tissue distributions. The main scan pulse Pₘ is not limited to pulse excitation of a single frequency, and a pulse excitation waveform (such as a Chirp wave using a linear frequency modulation technology) containing multiple frequencies can be considered. Therefore, the Pₘ covers all the scan frequencies which can be supported by the system, and thus, the echo generated by the Pₘ also contains various tissue information of different frequency bands, thereby being beneficial to subsequently better confirming selection of the supplementary scan pulse in an echo real-time identification system.

For example, for the supplementary scan pulses Pₓ₁, Pₓ₂, or the like, a plurality of different supplementary scan pulse waveforms are pre-stored in the computer device, and during each scan, the computer device determines which supplementary scan pulses need to be additionally transmitted according to the main scan pulse echo identification result. The specific supplementary scan pulse may be a pulse excitation signal of a single frequency, or a modulated pulse signal covering multiple frequencies.

In this embodiment, the main scan pulse echo and the at least one supplementary scan pulse echo are taken as the group of single-scan data corresponding to the target position; the plurality of different target positions are determined and the group of single-scan data corresponding to each target position is acquired; and the intravascular ultrasound imaging result is obtained according to the multiple groups of single-scan data. The supplementary scan pulse can have the scan frequency different from that of the main scan pulse, and ultrasound imaging is performed in combination with multi-frequency scan pulses, so that clear and complete images can be ensured to be obtained in different intravascular scenarios, and applicability of intravascular ultrasound imaging is improved.

In an embodiment, the step of obtaining the intravascular ultrasound imaging result according to the multiple groups of single-scan data may include: respectively performing information fusion on the main scan pulse echo and the at least one supplementary scan pulse echo in the same group of single-scan data to obtain a plurality of fusion echo signals; and performing image reconstruction according to the plurality of fusion echo signals to obtain the intravascular ultrasound imaging result.

In a specific embodiment, a number of the echoes received for each scan is not certain due to uncertainty of the supplementary scan pulse, and the number of the echoes for each scan is equal to a sum of a number of the main pulse scan echoes and a number of the supplementary scan pulse echoes. For IVUS image reconstruction, however, only one echo signal is required for each scan. As shown in FIG. 5, information fusion may be first performed on the plurality of echo signals belonging to one scan, and the echo signals may be fused into one ultrasonic echo signal by linear weighted fusion or based on an image fusion algorithm in other transform domains. Then, energy matching and normalization are performed on the fusion ultrasonic echo signals belonging to different scans. Finally, image reconstruction is performed on the fusion ultrasonic echo signals belonging to the same image frame, and the IVUS image with richer and more accurate tissue information can be obtained.

In this embodiment, the information fusion is performed on the main scan pulse echo and the at least one supplementary scan pulse echo in the same group of single-scan data to obtain the plurality of fusion echo signals, and the image reconstruction is performed according to the plurality of fusion echo signals to obtain the intravascular ultrasound imaging result. Ultrasound imaging can be performed in combination with multi-frequency scan pulses, and the applicability of intravascular ultrasound imaging is improved.

In an embodiment, the step of obtaining the intravascular ultrasound imaging result according to the multiple groups of single-scan data may further include: performing image reconstruction according to the main scan pulse echo in each group of single-scan data to obtain a first imaging result; performing image reconstruction according to the at least one supplementary scan pulse echo in each group of single-scan data to obtain at least one second imaging result; and performing image fusion according to the first imaging result and the at least one second imaging result to obtain the intravascular ultrasound imaging result.

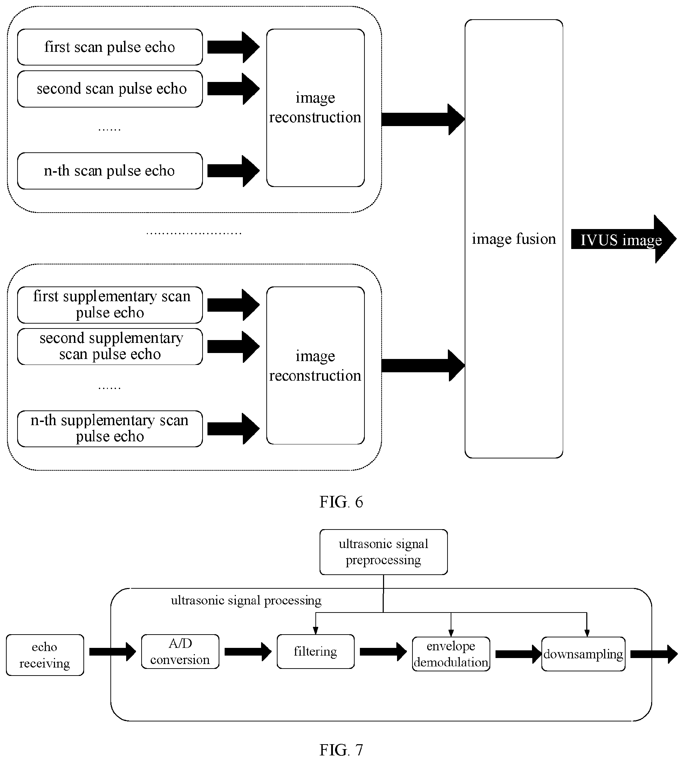

In an embodiment, as shown in FIG. 6, the main scan pulse echo and the supplementary scan pulse echo obtained by each scan may be processed separately. That is, IVUS image reconstruction is performed on the main scan pulse echo and the supplementary scan pulse echo, and reconstructed images are then displayed simultaneously or in a fusion manner. In actual operation, the IVUS image reconstructed using the main scan pulse echo is used as main display to be played and displayed in real time. If the IVUS image of the supplementary scan pulse still exists in a current IVUS scan frame, it can be displayed synchronously beside the IVUS image of the main scan pulse, or the IVUS images (the IVUS image of the main scan pulse and the IVUS image of the supplementary scan pulse) at the same moment can be subjected to real-time fusion, including but not limited to weighted linear fusion or image fusion in other transform domains, and then displayed.

In this embodiment, image reconstruction is performed according to the main scan pulse echo in each group of single-scan data to obtain the first imaging result; image reconstruction is performed according to the at least one supplementary scan pulse echo in each group of single-scan data to obtain the at least one second imaging result; and image fusion is performed according to the first imaging result and the at least one second imaging result to obtain the intravascular ultrasound imaging result. Ultrasound imaging can be performed in combination with multi-frequency scan pulses, and the applicability of intravascular ultrasound imaging is improved.

In an embodiment, the step of according to the main scan pulse echo and the at least one supplementary scan pulse echo, obtaining an intravascular ultrasound imaging result may further include: acquiring a signal frequency of the main scan pulse echo; determining preprocessing parameters according to the signal frequency, and performing ultrasonic signal preprocessing on the main scan pulse echo and the at least one supplementary scan pulse echo by adopting the preprocessing parameters; and obtaining the intravascular ultrasound imaging result according to the main scan pulse echo and the at least one supplementary scan pulse echo after the ultrasonic signal preprocessing.

In an embodiment, preset preprocessing parameters corresponding to different scan signal frequencies exist in the computer device and include, but are not limited to, a tap coefficient of an FIR band-pass filter, a demodulation coefficient in quadrature demodulation, or the like. Each time the IVUS device emits the main scan pulse, the computer device identifies a signal frequency of the main scan pulse, equates this signal frequency to the signal frequency of the main scan pulse echo, and then automatically configures the preprocessing parameters matching the signal frequency to ensure that ultrasonic echo information of different signal frequencies can be processed using the correct parameters.

Specifically, a flow of performing ultrasonic signal preprocessing on the main scan pulse echo and the at least one supplementary scan pulse echo by adopting the preprocessing parameters is shown in FIG. 7.

A/D conversion is used to convert an analog ultrasonic signal transmitted from an echo receiving circuit into a digital ultrasonic signal.

Filtering is used to denoise the digital ultrasonic signal to improve a signal-to-noise ratio of the ultrasonic signal. Specific filtering methods include, but are not limited to, FIR filtering, IIR filtering, wavelet decomposition, or the like. Taking FIR filtering as an example, an FIR filter processing program is implemented, but the FIR tap coefficient is not directly solidified in a code, and the corresponding FIR tap coefficient is configured in real time according to the current scan frequency.

Envelope demodulation is used for quadrature demodulation, Hilbert transform, or the like. The ultrasonic signal emitted by a transducer is reflected when passing through the vascular tissue, and the ultrasonic echo signal is generated. The echo signal has the same oscillation frequency as the emitted ultrasonic signal, and tissue-related information is modulated in the echo signal in a manner similar to amplitude modulation. Therefore, in order to extract corresponding tissue information from the ultrasonic echo signal, envelope demodulation is required to be performed on the ultrasonic echo signal. Taking quadrature demodulation as an example, an orthogonal demodulation algorithm is adopted, but a value of a center frequency for the quadrature demodulation algorithm is not solidified in the program, but is configured in real time according to the current scan frequency.

Downsampling may be used to reduce a sampling rate according to the Nyquist sampling theorem, thereby reducing an amount of data required to be processed to improve a subsequent image processing efficiency. A frequency range of the related information of the vascular tissue of the ultrasonic signal after envelope demodulation can be reduced to a lower frequency range, and the reduction of the sampling rate may reduce the amount of the data required to be processed to improve the subsequent image processing efficiency. Before downsampling, a low-pass filter is generally required to perform low-pass filtering to remove high-frequency interference components. The low-pass filter may need to be changed due to the difference of the scan center frequency, and therefore, a filter coefficient of the filter is also set in real time according to the current scan frequency.

In this embodiment, the signal frequency of the main scan pulse echo is acquired; the preprocessing parameters are determined according to the signal frequency, and ultrasonic signal preprocessing is performed on the main scan pulse echo and the at least one supplementary scan pulse echo by adopting the preprocessing parameters; and the intravascular ultrasound imaging result is obtained according to the main scan pulse echo and the at least one supplementary scan pulse echo after the ultrasonic signal preprocessing. The echo signal can be preprocessed according to the preprocessing parameter determined by the signal frequency, processing of the multi-frequency scan pulses is realized, ultrasound imaging is performed in combination with the multi-frequency scan pulses, and the applicability of intravascular ultrasound imaging is improved.

In an embodiment, the step of obtaining waveform feature-supplementary pulse type association information may include: acquiring a plurality of vascular tissue features related to intravascular ultrasound, and determining a supplementary pulse type corresponding to each vascular tissue feature; acquiring a sample scan pulse echo corresponding to each vascular tissue feature, and extracting a sample waveform feature corresponding to each vascular tissue feature from each sample scan pulse echo; and acquiring the waveform feature-supplementary pulse type association information according to the sample waveform feature and the supplementary pulse type corresponding to the same vascular tissue feature.

In an embodiment, the vascular tissue feature includes the distance between the vascular tissue and the probe, the thickness of the vascular tissue, or the like. For example, for a case where the vascular tissue is closer to the probe or the tissue thickness is smaller, a high frequency supplementary pulse type may be selected because high frequency pulse excitation has a higher axial resolution and a smaller penetration depth, thereby obtaining more details of the vascular tissue. For a case where the vascular tissue is further from the probe or thicker, a low frequency supplementary pulse type with higher penetration power is used. Similarly, through the waveform feature of the high-energy band echo, the problems such as calcification regions or guide wire artifacts can be identified in the echo through the similar method, so as to determine the target supplementary pulse type.

In this embodiment, the plurality of vascular tissue features related to intravascular ultrasound are acquired, and the supplementary pulse type corresponding to each vascular tissue feature is determined; the sample scan pulse echo corresponding to each vascular tissue feature is acquired, and the sample waveform feature corresponding to each vascular tissue feature is extracted from each sample scan pulse echo; and the waveform feature-supplementary pulse type association information can be obtained according to the sample waveform feature and the supplementary pulse type corresponding to the same vascular tissue feature, so that in each scan process, the proper supplementary scan pulse is automatically matched according to the identification result of the echo, and an efficiency of intravascular ultrasound imaging is improved.

In an embodiment, the step of extracting a waveform feature of the main scan pulse echo, and according to waveform feature-supplementary pulse type association information, determining a target supplementary pulse type corresponding to the waveform feature of the main scan pulse echo may include: identifying the waveform feature to obtain an identification result; if the identification result meets a supplementary pulse condition, determining the target supplementary pulse type corresponding to the waveform feature according to the waveform feature-supplementary pulse type association information; and if the identification result does not meet the supplementary pulse condition, obtaining the intravascular ultrasound imaging result according to the main scan pulse echo.

Specifically, the computer device identifies the vascular tissue distribution information in real time through the main scan pulse echo, the vascular tissue distribution information is equivalent to the waveform feature, if the problems that the distance between the vascular tissue and the IVUS probe is large, and the thickness of the vascular tissue is large exist in the vascular tissue distribution information, the identification result of the waveform feature is considered to meet the supplementary pulse condition, and the target supplementary pulse type corresponding to the waveform feature is selected according to the preset waveform feature-supplementary pulse type association information. If the problems that the distance between the vascular tissue and the IVUS probe is large, and the thickness of the vascular tissue is large do not exist in the vascular tissue distribution information, the identification result of the waveform feature is considered not to meet the supplementary pulse condition, the supplementary scan pulse does not need to be emitted again, and the main scan pulse echo can be directly adopted for intravascular ultrasound imaging.

In this embodiment, the waveform feature is identified to obtain the identification result; and if the identification result meets the supplementary pulse condition, the target supplementary pulse type corresponding to the waveform feature is determined according to the waveform feature-supplementary pulse type association information. If the identification result does not meet the supplementary pulse condition, the intravascular ultrasound imaging result is obtained according to the main scan pulse echo. Only when the identification result of the echo meets the supplementary pulse condition, the supplementary scan pulse is continuously emitted, so that the condition that the supplementary scan pulse is still continuously emitted when the supplementary scan pulse is not required is avoided, and the efficiency of the intravascular ultrasound imaging is improved.

In an embodiment, an intravascular ultrasound imaging method includes the following steps:
acquiring a plurality of vascular tissue features related to intravascular ultrasound, and determining a supplementary pulse type corresponding to each vascular tissue feature; acquiring a sample scan pulse echo corresponding to each vascular tissue feature, and extracting a sample waveform feature corresponding to each vascular tissue feature from each sample scan pulse echo; and acquiring waveform feature-supplementary pulse type association information according to the sample waveform feature and the supplementary pulse type corresponding to the same vascular tissue feature;
emitting a main scan pulse to a target position, and receiving a returned main scan pulse echo;
extracting the waveform feature of the main scan pulse echo, and identifying the waveform feature to obtain an identification result; if the identification result does not meet a supplementary pulse condition, directly obtaining an intravascular ultrasound imaging result according to the main scan pulse echo; if the identification result meets the supplementary pulse condition, determining a target supplementary pulse type corresponding to the waveform feature according to the waveform feature-supplementary pulse type association information;
determining at least one supplementary scan pulse according to the target supplementary pulse type, emitting the at least one supplementary scan pulse to the target position at one time, and receiving returned supplementary scan pulse echoes in sequence to obtain the supplementary scan pulse echoes respectively corresponding to the supplementary scan pulses;
acquiring a signal frequency of the main scan pulse echo; determining preprocessing parameters according to the signal frequency, and performing ultrasonic signal preprocessing on the main scan pulse echo and the at least one supplementary scan pulse echo by adopting the preprocessing parameters;
taking the preprocessed main scan pulse echo and at least one supplementary scan pulse echo as a group of single-scan data corresponding to the target position; determining a plurality of different target positions in a scan period, and acquiring the group of single-scan data corresponding to each target position to obtain a plurality of groups of single-scan data in the scan period;
respectively performing information fusion on the main scan pulse echo and the at least one supplementary scan pulse echo in the same group of single-scan data to obtain a plurality of fusion echo signals; and performing image reconstruction according to the plurality of fusion echo signals to obtain the intravascular ultrasound imaging result; or
performing image reconstruction according to the main scan pulse echo in each group of single-scan data to obtain a first imaging result; performing image reconstruction according to the at least one supplementary scan pulse echo in each group of single-scan data to obtain at least one second imaging result; and performing image fusion according to the first imaging result and the at least one second imaging result to obtain the intravascular ultrasound imaging result.

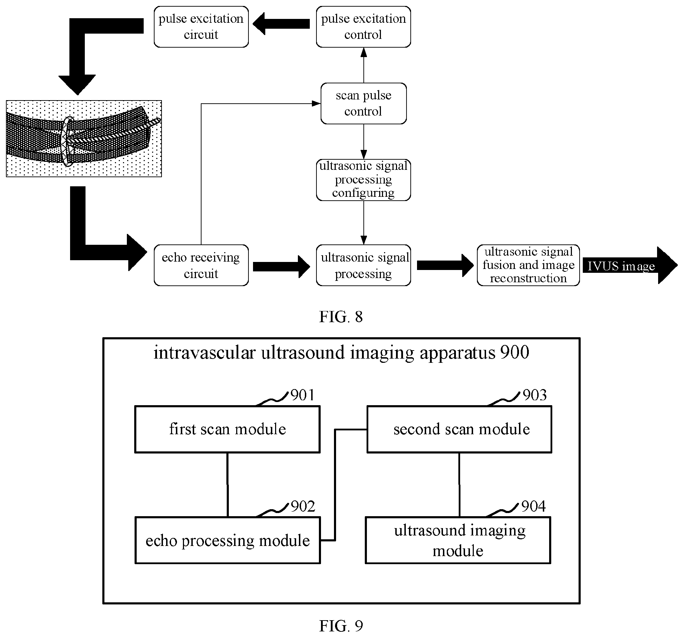

In an embodiment, with the intravascular ultrasound imaging method applied to an intravascular ultrasound imaging system as an example, as shown in FIG. 8, the system includes: a scan pulse control module, a pulse excitation control module, a pulse excitation circuit, an echo receiving circuit, an ultrasonic signal processing configuring module, an ultrasonic signal processing module and a signal fusion and image reconstruction module.

Specifically, the scan pulse control module is configured to determine the scan pulses (the main scan pulse and the supplementary scan pulse) that should be currently used, and send corresponding control information to the pulse excitation control module and the ultrasonic signal processing configuring module in real time.

The pulse excitation control module is configured to generate a corresponding pulse control signal in real time according to a control signal of the scan frequency control module, so that the pulse excitation circuit is controlled to emit corresponding ultrasonic pulse excitation.

The pulse excitation circuit is configured to be controlled by the pulse excitation control module to generate a pulse excitation signal in a range of 20-80MHz or a wider range to excite the IVUS ultrasound probe, so as to generate an ultrasonic wave.

The echo receiving circuit is configured to receive an electric signal obtained by converting an ultrasonic echo signal through the IVUS probe and perform corresponding processing (such as small signal amplification and anti-aliasing filtering).

The ultrasonic signal processing configuring module is configured to generate corresponding signal processing related parameters (such as band-pass filter coefficients) in real time according to a control signal of the scan frequency control module and send the corresponding signal processing related parameters to the ultrasonic signal processing module in real time.

The ultrasonic signal processing module is configured to convert the ultrasonic signal transmitted by the echo receiving circuit from an analog signal to a digital signal and then perform corresponding digital signal processing.

The signal fusion and image reconstruction module is configured to receive the ultrasonic signal processed by the ultrasonic signal processing module and perform fusion and ultrasonic reconstruction on echoes corresponding to different scan pulses, so as to obtain the two-dimensional IVUS image.

It should be understood that, although the steps in the flow charts involved in the above embodiments are shown in sequence as indicated by the arrows, the steps are not necessarily performed in sequence as indicated by the arrows. Unless explicitly stated herein, the steps are not limited to being performed in the exact order and may be performed in other orders. At least part of the steps in the flow charts involved in the above embodiments may include multiple steps or multiple stages, which are not necessarily performed at the same moment, but may be performed at different moments, and the steps or the stages are not necessarily performed in sequence, but may be performed alternately with other steps or at least part of the steps or the stages in other steps.

Based on the same inventive concept, the embodiment of the present application further provides an intravascular ultrasound imaging apparatus for implementing the above intravascular ultrasound imaging method. The implementation solution for solving problems provided by the apparatus is similar to the implementation solution described in the above method, and therefore, for the specific definitions in one or more embodiments of the intravascular ultrasound imaging apparatus provided below, reference can be made to the definitions of the intravascular ultrasound imaging method in the above description, and the definitions are not repeated herein.

In an embodiment, as shown in FIG. 9, there is provided an intravascular ultrasound imaging apparatus 900, including:
a first scan module 901 configured to acquire a main scan pulse echo corresponding to a main scan pulse;
an echo processing module 902 configured to extract a waveform feature of the main scan pulse echo, obtain waveform feature-supplementary pulse type association information, and according to the association information, determine a target supplementary pulse type corresponding to the waveform feature of the main scan pulse echo;
a second scan module 903 configured to, according to the target supplementary pulse type, determine at least one supplementary scan pulse, and acquire supplementary scan pulse echoes respectively corresponding to the supplementary scan pulses; and
an ultrasound imaging module 904 configured to, according to the main scan pulse echo and the at least one supplementary scan pulse echo, obtain an intravascular ultrasound imaging result.

In an embodiment, the first scan module 901 is further configured to emit the main scan pulse to a target position, and receive the returned main scan pulse echo.

In an embodiment, the second scan module 903 is further configured to emit the at least one supplementary scan pulse to the target position at one time, and receive the returned supplementary scan pulse echoes in sequence to obtain the at least one supplementary scan pulse echo corresponding to the at least one supplementary scan pulse respectively.

In an embodiment, the ultrasound imaging module 904 is further configured to: take the main scan pulse echo and the at least one supplementary scan pulse echo as a group of single-scan data corresponding to the target position; in a scan period, determine a plurality of different target positions and acquire the group of single-scan data corresponding to each target position; and obtain the intravascular ultrasound imaging result according to the multiple groups of single-scan data.

In an embodiment, the ultrasound imaging module 904 is further configured to: respectively perform information fusion on the main scan pulse echo and the at least one supplementary scan pulse echo in the same group of single-scan data to obtain a plurality of fusion echo signals; and perform image reconstruction according to the plurality of fusion echo signals to obtain the intravascular ultrasound imaging result.

In an embodiment, the ultrasound imaging module 904 is further configured to: perform image reconstruction according to the main scan pulse echo in each group of single-scan data to obtain a first imaging result; perform image reconstruction according to the at least one supplementary scan pulse echo in each group of single-scan data to obtain at least one second imaging result; and perform image fusion according to the first imaging result and the at least one second imaging result to obtain the intravascular ultrasound imaging result.

In an embodiment, the apparatus further includes:
a signal processing module configured to: acquire a signal frequency of the main scan pulse echo; determine preprocessing parameters according to the signal frequency, and perform ultrasonic signal preprocessing on the main scan pulse echo and the at least one supplementary scan pulse echo by adopting the preprocessing parameters; and obtain the intravascular ultrasound imaging result according to the main scan pulse echo and the at least one supplementary scan pulse echo after the ultrasonic signal preprocessing.

In an embodiment, the echo processing module 902 is further configured to: acquire a plurality of vascular tissue features related to intravascular ultrasound, and determine a supplementary pulse type corresponding to each vascular tissue feature; acquire a sample scan pulse echo corresponding to each vascular tissue feature, and extract a sample waveform feature corresponding to each vascular tissue feature from each sample scan pulse echo; and acquire the waveform feature-supplementary pulse type association information according to the sample waveform feature and the supplementary pulse type corresponding to the same vascular tissue feature.

In an embodiment, the echo processing module 902 is further configured to: identify the waveform feature to obtain an identification result; if the identification result meets a supplementary pulse condition, determine the target supplementary pulse type corresponding to the waveform feature according to the waveform feature-supplementary pulse type association information.

In an embodiment, the ultrasound imaging module 904 is further configured to: if the identification result does not meet the supplementary pulse condition, obtain the intravascular ultrasound imaging result according to the main scan pulse echo.

The modules in the intravascular ultrasound imaging apparatus may be wholly or partially implemented by software, hardware and a combination thereof. The modules may be embedded in or independent of the processor in the computer device in hardware, or may be stored in the memory in the computer device in software, such that the processor can conveniently call the modules to execute the operations corresponding to the modules.

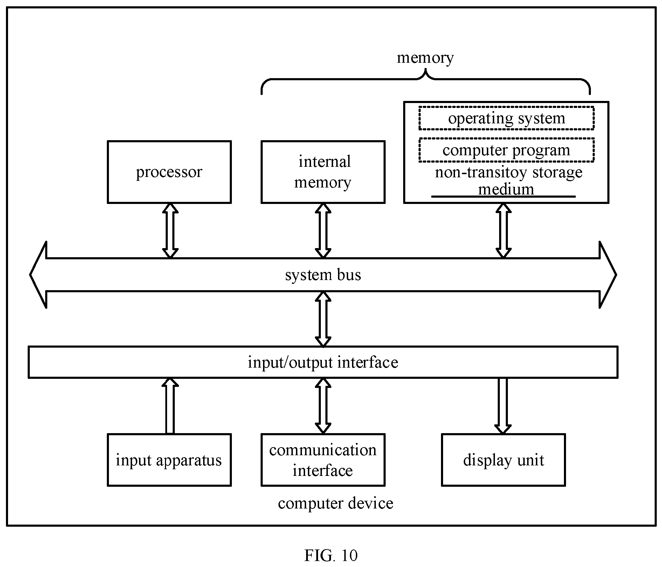

In an embodiment, there is provided a computer device, which may be a terminal, an internal structure diagram of which may be shown in FIG. 10. The computer device includes a processor, a memory, an input/output interface, a communication interface, a display unit, and an input apparatus. The processor, the memory, and the input/output interface are connected through a system bus, and the communication interface, the display unit, and the input apparatus are connected to the system bus through the input/output interface. The processor of the computer device is configured to provide computing and control capabilities. The memory of the computer device includes a non-transitory storage medium and an internal memory. The non-transitory storage medium stores an operating system and a computer program. The internal memory provides an environment for running of the operating system and the computer program in the non-transitory storage medium. The input/output interface of the computer device is configured to exchange information between the processor and an external device. The communication interface of the computer device is configured to be communicated with an external terminal in a wired or wireless mode, and the wireless mode can be implemented through WIFI, a mobile cellular network, NFC (near field communication), or other technologies. The computer program is executed by the processor to implement an intravascular ultrasound imaging method. The display unit of the computer device is configured to form a visually visible picture and can be a display screen, a projection apparatus, or a virtual reality imaging apparatus. The display screen may be a liquid crystal display screen or an electronic ink display screen, and the input apparatus of the computer device may be a touch layer covering the display screen, or may be a key, a trackball or a touch pad arranged on a casing of the computer device, or may be an external keyboard, touch pad, mouse, or the like.

Those skilled in the art will appreciate that the structure shown in FIG. 10 is only a block diagram of a part of the structure associated with the application solution and does not constitute a limitation on the computer device to which the application solution is applied, and a particular computer device may include more or fewer components than shown components, or combine certain components, or have a different arrangement of components.

In an embodiment, there is provided a computer device, including a memory and a processor, the memory storing a computer program, and the processor implementing the following steps when executing the computer program: acquiring a main scan pulse echo corresponding to a main scan pulse; extracting a waveform feature of the main scan pulse echo, and according to waveform feature-supplementary pulse type association information, determining a target supplementary pulse type corresponding to the waveform feature of the main scan pulse echo; according to the target supplementary pulse type, determining at least one supplementary scan pulse, and acquiring supplementary scan pulse echoes respectively corresponding to the supplementary scan pulses; and according to the main scan pulse echo and the at least one supplementary scan pulse echo, obtaining an intravascular ultrasound imaging result.

In an embodiment, the processor also implements the following steps when executing the computer program: emitting the main scan pulse to a target position, and receiving the returned main scan pulse echo.

In an embodiment, the processor also implements the following steps when executing the computer program: emitting the at least one supplementary scan pulse to the target position at one time, and receiving the returned supplementary scan pulse echoes in sequence to obtain the at least one supplementary scan pulse echo corresponding to the at least one supplementary scan pulse respectively.

In an embodiment, the processor also implements the following steps when executing the computer program: taking the main scan pulse echo and the at least one supplementary scan pulse echo as a group of single-scan data corresponding to the target position; in a scan period, determining a plurality of different target positions and acquire the group of single-scan data corresponding to each target position; and obtaining the intravascular ultrasound imaging result according to the multiple groups of single-scan data.

In an embodiment, the processor also implements the following steps when executing the computer program: respectively performing information fusion on the main scan pulse echo and the at least one supplementary scan pulse echo in the same group of single-scan data to obtain a plurality of fusion echo signals; and performing image reconstruction according to the plurality of fusion echo signals to obtain the intravascular ultrasound imaging result.

In an embodiment, the processor also implements the following steps when executing the computer program: performing image reconstruction according to the main scan pulse echo in each group of single-scan data to obtain a first imaging result; performing image reconstruction according to the at least one supplementary scan pulse echo in each group of single-scan data to obtain at least one second imaging result; and performing image fusion according to the first imaging result and the at least one second imaging result to obtain the intravascular ultrasound imaging result.

In an embodiment, the processor also implements the following steps when executing the computer program: acquiring a signal frequency of the main scan pulse echo; determining preprocessing parameters according to the signal frequency, and performing ultrasonic signal preprocessing on the main scan pulse echo and the at least one supplementary scan pulse echo by adopting the preprocessing parameters; and obtaining the intravascular ultrasound imaging result according to the main scan pulse echo and the at least one supplementary scan pulse echo after the ultrasonic signal preprocessing.

In an embodiment, the processor also implements the following steps when executing the computer program: acquiring a plurality of vascular tissue features related to intravascular ultrasound, and determining a supplementary pulse type corresponding to each vascular tissue feature; acquiring a sample scan pulse echo corresponding to each vascular tissue feature, and extracting a sample waveform feature corresponding to each vascular tissue feature from each sample scan pulse echo; and acquiring the waveform feature-supplementary pulse type association information according to the sample waveform feature and the supplementary pulse type corresponding to the same vascular tissue feature.

In an embodiment, the processor also implements the following steps when executing the computer program: identifying the waveform feature to obtain an identification result; if the identification result meets a supplementary pulse condition, determining the target supplementary pulse type corresponding to the waveform feature according to the waveform feature-supplementary pulse type association information.

In an embodiment, the processor also implements the following step when executing the computer program: if the identification result does not meet the supplementary pulse condition, obtaining the intravascular ultrasound imaging result according to the main scan pulse echo.

In an embodiment, there is provided a computer-readable storage medium having a computer program stored thereon, the computer program, when executed by a processor, implementing the following steps: acquiring a main scan pulse echo corresponding to a main scan pulse; extracting a waveform feature of the main scan pulse echo, and according to waveform feature-supplementary pulse type association information, determining a target supplementary pulse type corresponding to the waveform feature of the main scan pulse echo; according to the target supplementary pulse type, determining at least one supplementary scan pulse, and acquiring supplementary scan pulse echoes respectively corresponding to the supplementary scan pulses; and according to the main scan pulse echo and the at least one supplementary scan pulse echo, obtaining an intravascular ultrasound imaging result.

In an embodiment, the computer program, when executed by the processor, also implements the following steps: emitting the main scan pulse to a target position, and receiving the returned main scan pulse echo.

In an embodiment, the computer program, when executed by the processor, also implements the following steps: emitting the at least one supplementary scan pulse to the target position at one time, and receiving the returned supplementary scan pulse echoes in sequence to obtain the at least one supplementary scan pulse echo corresponding to the at least one supplementary scan pulse respectively.

In an embodiment, the computer program, when executed by the processor, also implements the following steps: taking the main scan pulse echo and the at least one supplementary scan pulse echo as a group of single-scan data corresponding to the target position; in a scan period, determining a plurality of different target positions and acquire the group of single-scan data corresponding to each target position; and obtaining the intravascular ultrasound imaging result according to the multiple groups of single-scan data.

In an embodiment, the computer program, when executed by the processor, also implements the following steps: respectively performing information fusion on the main scan pulse echo and the at least one supplementary scan pulse echo in the same group of single-scan data to obtain a plurality of fusion echo signals; and performing image reconstruction according to the plurality of fusion echo signals to obtain the intravascular ultrasound imaging result.

In an embodiment, the computer program, when executed by the processor, also implements the following steps: performing image reconstruction according to the main scan pulse echo in each group of single-scan data to obtain a first imaging result; performing image reconstruction according to the at least one supplementary scan pulse echo in each group of single-scan data to obtain at least one second imaging result; and performing image fusion according to the first imaging result and the at least one second imaging result to obtain the intravascular ultrasound imaging result.

In an embodiment, the computer program, when executed by the processor, also implements the following steps: acquiring a signal frequency of the main scan pulse echo; determining preprocessing parameters according to the signal frequency, and performing ultrasonic signal preprocessing on the main scan pulse echo and the at least one supplementary scan pulse echo by adopting the preprocessing parameters; and obtaining the intravascular ultrasound imaging result according to the main scan pulse echo and the at least one supplementary scan pulse echo after the ultrasonic signal preprocessing.

In an embodiment, the computer program, when executed by the processor, also implements the following steps: acquiring a plurality of vascular tissue features related to intravascular ultrasound, and determining a supplementary pulse type corresponding to each vascular tissue feature; acquiring a sample scan pulse echo corresponding to each vascular tissue feature, and extracting a sample waveform feature corresponding to each vascular tissue feature from each sample scan pulse echo; and acquiring the waveform feature-supplementary pulse type association information according to the sample waveform feature and the supplementary pulse type corresponding to the same vascular tissue feature.

In an embodiment, the computer program, when executed by the processor, also implements the following steps: identifying the waveform feature to obtain an identification result; if the identification result meets a supplementary pulse condition, determining the target supplementary pulse type corresponding to the waveform feature according to the waveform feature-supplementary pulse type association information.

In an embodiment, the computer program, when executed by the processor, also implements the following step: if the identification result does not meet the supplementary pulse condition, obtaining the intravascular ultrasound imaging result according to the main scan pulse echo.

In an embodiment, there is provided a computer program product, including a computer program, the computer program, when executed by a processor, implementing the following steps: acquiring a main scan pulse echo corresponding to a main scan pulse; extracting a waveform feature of the main scan pulse echo, and according to waveform feature-supplementary pulse type association information, determining a target supplementary pulse type corresponding to the waveform feature of the main scan pulse echo; according to the target supplementary pulse type, determining at least one supplementary scan pulse, and acquiring supplementary scan pulse echoes respectively corresponding to the supplementary scan pulses; and according to the main scan pulse echo and the at least one supplementary scan pulse echo, obtaining an intravascular ultrasound imaging result.

In an embodiment, the computer program, when executed by the processor, also implements the following steps: emitting the main scan pulse to a target position, and receiving the returned main scan pulse echo.

In an embodiment, the computer program, when executed by the processor, also implements the following steps: emitting the at least one supplementary scan pulse to the target position at one time, and receiving the returned supplementary scan pulse echoes in sequence to obtain the at least one supplementary scan pulse echo corresponding to the at least one supplementary scan pulse respectively.

In an embodiment, the computer program, when executed by the processor, also implements the following steps: taking the main scan pulse echo and the at least one supplementary scan pulse echo as a group of single-scan data corresponding to the target position; in a scan period, determining a plurality of different target positions and acquire the group of single-scan data corresponding to each target position; and obtaining the intravascular ultrasound imaging result according to the multiple groups of single-scan data.

In an embodiment, the computer program, when executed by the processor, also implements the following steps: respectively performing information fusion on the main scan pulse echo and the at least one supplementary scan pulse echo in the same group of single-scan data to obtain a plurality of fusion echo signals; and performing image reconstruction according to the plurality of fusion echo signals to obtain the intravascular ultrasound imaging result.

In an embodiment, the computer program, when executed by the processor, also implements the following steps: performing image reconstruction according to the main scan pulse echo in each group of single-scan data to obtain a first imaging result; performing image reconstruction according to the at least one supplementary scan pulse echo in each group of single-scan data to obtain at least one second imaging result; and performing image fusion according to the first imaging result and the at least one second imaging result to obtain the intravascular ultrasound imaging result.

In an embodiment, the computer program, when executed by the processor, also implements the following steps: acquiring a signal frequency of the main scan pulse echo; determining preprocessing parameters according to the signal frequency, and performing ultrasonic signal preprocessing on the main scan pulse echo and the at least one supplementary scan pulse echo by adopting the preprocessing parameters; and obtaining the intravascular ultrasound imaging result according to the main scan pulse echo and the at least one supplementary scan pulse echo after the ultrasonic signal preprocessing.

In an embodiment, the computer program, when executed by the processor, also implements the following steps: acquiring a plurality of vascular tissue features related to intravascular ultrasound, and determining a supplementary pulse type corresponding to each vascular tissue feature; acquiring a sample scan pulse echo corresponding to each vascular tissue feature, and extracting a sample waveform feature corresponding to each vascular tissue feature from each sample scan pulse echo; and acquiring the waveform feature-supplementary pulse type association information according to the sample waveform feature and the supplementary pulse type corresponding to the same vascular tissue feature.

In an embodiment, the computer program, when executed by the processor, also implements the following steps: identifying the waveform feature to obtain an identification result; if the identification result meets a supplementary pulse condition, determining the target supplementary pulse type corresponding to the waveform feature according to the waveform feature-supplementary pulse type association information.

In an embodiment, the computer program, when executed by the processor, also implements the following step: if the identification result does not meet the supplementary pulse condition, obtaining the intravascular ultrasound imaging result according to the main scan pulse echo.

It should be noted that user information (including but not limited to user device information, user personal information, or the like) and data (including but not limited to data for analysis, stored data, displayed data, or the like) involved in the present application are information and data authorized by the user or sufficiently authorized by each party, and collection, use and processing of relevant data require compliance with relevant laws, regulations and standards in relevant countries and regions.

It will be understood by those skilled in the art that all or part of the processes of the method according to the embodiments described above may be implemented by a computer program instructing related hardware, and the computer program may be stored in a non-transitory computer-readable storage medium, and when executed, may include the processes of the embodiments of the method described above. Any reference to memories, databases or other media used in the embodiments of the present application can include at least one of a non-transitory memory and a transitory memory. The non-transitory memory may include a read-only memory (ROM), a magnetic tape, a floppy disk, a flash memory, an optical memory, a high-density embedded non-transitory memory, a resistive random access memory (ReRAM), a magnetoresistive random access memory (MRAM), a ferroelectric random access memory (FRAM), a phase change memory (PCM), a graphene memory, or the like. The transitory memory can include a random access memory (RAM), an external cache memory, or the like. By way of illustration and not limitation, the RAM can take many forms, such as a static random access memory (SRAM), a dynamic random access memory (DRAM), or the like. The databases involved in the embodiments of the present application may include at least one of relational and non-relational databases. The non-relational database may include, but is not limited to, a block chain-based distributed database, or the like. The processors referred to in the embodiments of the present application may include, but are not limited to, general processors, central processors, graphics processors, digital signal processors, programmable logic units, data processing logic units based on quantum calculations, or the like.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the specification.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the protection scope of the present application should be subject to the appended claims.

## Claims

1. An intravascular ultrasound imaging method, comprising:
acquiring a main scan pulse echo corresponding to a main scan pulse;
extracting a waveform feature of the main scan pulse echo, obtaining waveform feature-supplementary pulse type association information, and according to the association information, determining a target supplementary pulse type corresponding to the waveform feature of the main scan pulse echo;
according to the target supplementary pulse type, determining at least one supplementary scan pulse, and acquiring supplementary scan pulse echoes respectively corresponding to the supplementary scan pulses; and
according to the main scan pulse echo and the at least one supplementary scan pulse echo, obtaining an intravascular ultrasound imaging result.

2. The method according to claim 1, wherein acquiring the main scan pulse echo corresponding to the main scan pulse comprises:
emitting the main scan pulse to a target position, and receiving the returned main scan pulse echo.

3. The method according to claim 2, wherein acquiring the supplementary scan pulse echoes respectively corresponding to the supplementary scan pulses comprises:
emitting the at least one supplementary scan pulse to the target position at one time, and receiving the returned supplementary scan pulse echoes in sequence to obtain the at least one supplementary scan pulse echo.

4. The method according to claim 3, wherein according to the main scan pulse echo and the at least one supplementary scan pulse echo, obtaining the intravascular ultrasound imaging result comprises:
taking the main scan pulse echo and the at least one supplementary scan pulse echo as a group of single-scan data corresponding to the target position;
in a scan period, determining a plurality of different target positions and acquiring the group of single-scan data corresponding to each target position; and
obtaining the intravascular ultrasound imaging result according to the multiple groups of single-scan data.

5. The method according to claim 4, wherein obtaining the intravascular ultrasound imaging result according to the multiple groups of single-scan data comprises:
respectively performing information fusion on the main scan pulse echo and the at least one supplementary scan pulse echo in the same group of single-scan data to obtain a plurality of fusion echo signals; and
performing image reconstruction according to the plurality of fusion echo signals to obtain the intravascular ultrasound imaging result.

6. The method according to claim 4, wherein obtaining the intravascular ultrasound imaging result according to the multiple groups of single-scan data further comprises:
performing image reconstruction according to the main scan pulse echo in each group of single-scan data to obtain a first imaging result;
performing image reconstruction according to the at least one supplementary scan pulse echo in each group of single-scan data to obtain at least one second imaging result; and
performing image fusion according to the first imaging result and the at least one second imaging result to obtain the intravascular ultrasound imaging result.

7. The method according to claim 1, wherein according to the main scan pulse echo and the at least one supplementary scan pulse echo, obtaining the intravascular ultrasound imaging result further comprises:
acquiring a signal frequency of the main scan pulse echo;
determining preprocessing parameters according to the signal frequency, and performing ultrasonic signal preprocessing on the main scan pulse echo and the at least one supplementary scan pulse echo by adopting the preprocessing parameters; and
obtaining the intravascular ultrasound imaging result according to the main scan pulse echo and the at least one supplementary scan pulse echo after the ultrasonic signal preprocessing.

8. The method according to claim 1, wherein obtaining the waveform feature-supplementary pulse type association information comprises:
acquiring a plurality of vascular tissue features related to intravascular ultrasound, and determining a supplementary pulse type corresponding to each vascular tissue feature;
acquiring a sample scan pulse echo corresponding to each vascular tissue feature, and extracting a sample waveform feature corresponding to each vascular tissue feature from each sample scan pulse echo; and
acquiring the waveform feature-supplementary pulse type association information according to the sample waveform feature and the supplementary pulse type corresponding to the same vascular tissue feature.

9. The method according to claim 1, wherein extracting the waveform feature of the main scan pulse echo, obtaining the waveform feature-supplementary pulse type association information, and according to the association information, determining the target supplementary pulse type corresponding to the waveform feature of the main scan pulse echo comprises:
identifying the waveform feature to obtain an identification result; and
if the identification result meets a supplementary pulse condition, determining the target supplementary pulse type corresponding to the waveform feature according to the waveform feature-supplementary pulse type association information.

10. The method according to claim 9, further comprising:
if the identification result does not meet the supplementary pulse condition, obtaining the intravascular ultrasound imaging result according to the main scan pulse echo.

11. The method according to claim 1, wherein a scan frequency of the supplementary scan pulse is different from a scan frequency of the main scan pulse.

12. The method according to claim 7, wherein the preprocessing parameters comprise a tap coefficient of an FIR band-pass filter and a demodulation coefficient in quadrature demodulation.

13. An intravascular ultrasound imaging apparatus, comprising:
a first scan module configured to acquire a main scan pulse echo corresponding to a main scan pulse;
an echo processing module configured to extract a waveform feature of the main scan pulse echo, obtain waveform feature-supplementary pulse type association information, and according to the association information, determine a target supplementary pulse type corresponding to the waveform feature of the main scan pulse echo;
a second scan module further configured to, according to the target supplementary pulse type, determine at least one supplementary scan pulse, and acquire supplementary scan pulse echoes respectively corresponding to the supplementary scan pulses; and
an ultrasound imaging module configured to, according to the main scan pulse echo and the at least one supplementary scan pulse echo, obtain an intravascular ultrasound imaging result.

14. An intravascular ultrasound imaging system, comprising:
a scan pulse control module configured to determine scan pulses that should be currently used, and send corresponding control information to a pulse excitation control module and an ultrasonic signal processing configuring module;
the pulse excitation control module configured to control a pulse excitation circuit to emit corresponding ultrasonic pulse excitation according to the control information;
the pulse excitation circuit configured to be controlled by the pulse excitation control module to generate a pulse excitation signal to excite an IVUS ultrasound probe, so as to generate an ultrasonic wave;
an echo receiving circuit configured to receive an electric signal obtained after the IVUS probe converts an ultrasonic echo signal;
the ultrasonic signal processing configuring module configured to generate corresponding signal processing related parameters in real time according to the control signal and send the corresponding signal processing related parameters to an ultrasonic signal processing module;
the ultrasonic signal processing module configured to convert the electric signal received by the echo receiving circuit into a digital signal; and
a signal fusion and image reconstruction module configured to receive the digital signals generated by the ultrasonic signal processing module and perform fusion and ultrasonic reconstruction on the digital signals corresponding to different ultrasonic echo signals.

15. A computer device, comprising a memory and a processor, the memory storing a computer program, wherein the processor, when executing the computer program, implements steps of the method according to any one of claims 1 to 12.

16. A computer-readable storage medium having a computer program stored thereon, wherein the computer program, when executed by a processor, implements steps of the method according to any one of claims 1 to 12.
